# EUROPEAN PATENT SPECIFICATION

(11) **EP 4 725 465 B1**
(45) Date of publication and mention of the grant of the patent: **16.09.2026**
(21) Application number: 24205421.1
(22) Date of filing: 08.10.2024
(51) Int. Cl.: A61F 13/08, A61B 5/022, A61B 17/132, A61B 17/135, A61F 5/01

(54) **CUFF CONFIGURED TO BE CLOSED AROUND A LIMB FOR REDUCING OR INHIBITING A BLOOD FLOW**
MANSCHETTE ZUM VERSCHLIESSEN UM EIN GLIED ZUR REDUZIERUNG ODER HEMMUNG EINES BLUTFLUSSES
BRASSARD CONFIGURÉ POUR ÊTRE FERMÉ AUTOUR D'UN MEMBRE POUR RÉDUIRE OU INHIBER UN FLUX SANGUIN

(43) Date of publication of application: 15.04.2026
(73) Proprietor: Daisygrip GmbH, 18196 Kavelstorf (DE)
(72) Inventor: ALTRICHTER, Konstantin, 22303 Hamburg (DE); HARTMANN, Karl, 18107 Elmenhorst-Lichtenhagen (DE)
(74) Representative: Röthinger, Rainer

(56) References cited:
- DE-U- 1 998 179
- US-A- 4 727 885
- US-A1- 2020 297 352
- US-A1- 2023 270 448
- US-A1- 2023 355 245

## Description

### Technical Field

The present disclosure relates to a cuff that is configured to be closed around a limb for reducing or inhibiting a blood flow through the limb. Such cuffs can be used for blood pressure measurements, as constricting band or for similar purposes. The present disclosure also relates to methods for operating and disinfecting a cuff.

### Background

Cuffs for reducing or inhibiting the blood flow through a limb are well known in the art. Such cuffs have the shape of a band with a length that is sufficient to be wound around a limb, such as an arm or a leg. Once wound around the limb, the cuff has to be closed and maintain or withstand a pressure on the limb, and various closure mechanisms are known to this end. Many of these closure mechanisms are configured to be adjustable to the varying circumference of different persons' limbs.

Exemplary closure mechanisms comprise hook-and-loop fasteners of the Velcro type. Other closure mechanisms comprise a series of parallel metal bars sewn into or inserted into pockets of the cuff material and configured to be grasped by a hook attached to a free end of the cuff (see, e.g., German utility model DE 1 815 119). German utility model DE 1 998 179 explains that the latter closure mechanism is expensive and intricate, and that during extended use, the metal bars may penetrate the typically textile cuff material. To overcome these drawbacks, it is suggested in DE 1 998 179 to replace the metal bars by plastic webs that are interconnected by a thin plastic layer. Each of the plastic webs comprises a cavity configured to accommodate a hook that allows to securely close the cuff around a limb. However, threading the hook into a particular cavity can be cumbersome for a user, especially if the user applies the cuff to his or her own limb. Moreover, the tip of the hook can damage the plastic webs upon extended use or at high forces.

US patent application US 2023/027448 A1 discloses emergency tourniquets, and relates to a tourniquet system having an electronic tourniquet controller that may be used with several separate tourniquets on different persons. The tightening mechanism of an exemplary embodiment includes a strap extending from the first end 30 of the cuff and is removably insertable into the ratchet lock.

In the past decades, hygiene has become an increasingly important target in hospitals and among health professionals. The focus of many hygiene-related strategies is to keep medical devices free of contaminations, which is particularly challenging in case of re-usable medical devices such as cuffs.

Since (e.g., steam-based) sterilization devices are a scarce resource in hospitals, wipe disinfection is typically the solution of choice for allowing a safe re-use of cuffs. It has, however, been found that the hitherto known closure mechanisms are incompatible with a safe wipe disinfection. Evidently, hook-and-loop fasteners cannot be safely disinfected by simply wiping over the hooks and loops. Similar considerations apply to the closure mechanism of DE 1 998 179 because the cavities in the webs that are to accommodate the hooks define numerous hidden surfaces and edges that cannot easily be accessed by wiping.

### Summary

There is a need for a cuff that is configured to be closed around a limb for reducing or inhibiting a blood flow through the limb, and that avoids one or more of the drawbacks discussed above.

According to a first aspect, a cuff configured to be closed around a person's limb for reducing or inhibiting a blood flow through the limb is provided. The cuff comprises a flexible and substantially non-stretchable substrate defining a substrate length and a longitudinal axis extending along the substrate length. The cuff also comprises a first closure member attached to the substrate. The first closure member comprises a plurality of webs spaced apart from each other along the longitudinal axis of the substrate, with each web extending away from the substrate and defining a first abutment region extending over at least 50% of a length of the web. Furthermore, the cuff comprises a second closure member attached to the substrate at a free end thereof, wherein the second closure member defines a second abutment region configured to cooperate with the first abutment region of one of the webs of the first closure member to close the cuff around the limb. Further, the second closure member comprises two sections extending substantially parallel to each other and defining an opening there-between, wherein a first one of the two sections is pivotally attached to the substrate.

The webs may be continuous or non-continuous in their length direction. The first abutment region may be defined by a web surface extending away from the substrate. This surface may be planar or (e.g., convexly) curved. The first abutment region may extend over at least 75% of the length of each web or over the entire length of each web. The first abutment region may be provided at least at opposite ends of each web adjacent to the lateral sides of the substrate.

The first abutment region and the second abutment region may be configured for a blunt abutment. A blunt abutment may occur along a line or along an area. In some implementations, the blunt abutment may permit an at least limited relative movement of the first closure member and the second closure member in a direction extending along the length of the web, at least during an initial phase in which the cuff is applied to the limb and before a significant force is transmitted between the first abutment region and the second abutment region.

The first abutment region and the second abutment region may be configured with a closed smooth surface. The closed smooth surface may not comprise any openings for receiving one or more parts protruding from the complementary abutment region, such as hooks.

The second closure member may comprise an opening for threading the first closure member through the second closure member. The opening may have the form of an elongated slit. The cuff may permit the first abutment region and the second abutment region to come into abutment when first closure member is at least partially threaded through the opening of the second closure member. In the threaded state, the webs may be arranged on a side of the cuff facing away from the limb.

The opening in the second closure member may have a length dimension that exceeds a width dimension of the cuff in the region of the first closure member. The opening in the second closure member may have a width dimension that is slightly larger than a maximum thickness of the cuff in the region of the first closure member (as substantially defined by the height of the webs).

At least one of the first abutment region and the second abutment region may extend over at least 30%, and in particular over at least 50% (e.g., over 70% or more) of a width of at least one of the substrate and the cuff. In this way, a stable engagement of the abutment regions can be achieved.

A surface region of the cuff between two adjacent webs can be closed. It may in particular not include any openings for receiving components of a closure mechanism, such as hooks.

The first closure member may further comprise a planar basis extending at least between two adjacent webs. In some variants, the webs may be arranged on the planar basis (e.g., the webs may be welded to the basis or injection-molded together with the basis). The basis may be attached to the substrate. In some implementations, the basis defines the closed surface region of the cuff between two adjacent webs. The substrate may comprise a substrate window, and the basis may close the substrate window. In some cases, the basis may be attached to the substrate by ultrasound welding. In general, both the first and the second closure member may be attached to the substrate by welding, in particular ultrasound welding, in a gap-free manner to allow an easy disinfection.

The planar basis may have a rigidity that is low enough to allow an easy bending or flexing of the first closure member, but high enough to cause the first closure member to elastically assume its planar shape when a bending or flexing force is removed, in particular in a configuration in which the planar basis is attached to the substrate. Such a rigidity improves operability of the cuff and is particularly advantageous in an implementation in which the first closure member with the planar basis is threaded through an opening in the second closure member.

The substrate may be made from a fabric material. Additionally, or in the alternative, the first closure member may be made from a plastics material.

The second closure member comprises two sections extending substantially parallel to each other and defining an, or the, opening therebetween. The sections may have a linear extension (i.e., take the form of linear sections). A first one of the two sections, and thus the second closure member, is pivotally attached to the substrate. In use, the pivotably supported second closure member may rotate in a stable force-transmitting position in response to a pulling force acting thereon.

The second closure member may comprise (e.g., consist of) a loop or bracket. The loop or bracket may comprise the two linear sections and define the opening between the two linear sections.

A second one of the two sections may define the second abutment region. Alternatively, a second one of the two sections may pivotally support a closure element that defines the second abutment region. The closure element may comprise an undercut defining the second abutment region. The undercut may be defined by a groove in the closure element.

In some implementations, the webs may extend perpendicular to the longitudinal axis of the substrate. In other implementations, the webs may extend non-perpendicular to the longitudinal axis of the substrate (e.g., at an angle of over 45°).

The cuff may comprise an inflatable fluid chamber. The fluid chamber may be defined between two layers of the substrate material. The fluid chamber may be inflatable and deflatable. As such, it may used for blood pressure measurements, as is generally known in the art.

A further aspect relates to a method of operating the cuff presented herein. The method comprises threading the first closure member through the opening of the second closure member and inflating the fluid chamber to bring the first abutment region and the second abutment region into firm abutment. After the threading and before the inflation, the first abutment region and the second abutment region may have been brought into a pre-engagement. Depending on the situation, a pre-engagement may also comprise that the second closure member is trapped between two adjacent webs (e.g., can move only between an abutting relationship with either one of the two adjacent webs). The pre-engagement may allow an at least minor relative movement between the abutment regions along a length of a particular web.

Also presented herein is a method of disinfecting a cuff, in particular the cuff of the first aspect described herein, wherein the cuff comprises a substrate and two complementary closure members attached to the substrate by gap-free welds and configured to close the cuff by mutual abutment around a person's limb for reducing or inhibiting a blood flow through the limb, wherein the cuff has smooth surfaces (at least) in abutting regions of the closure members. The method comprises wiping over the cuff with a soft carrier of a disinfectant material.

The soft carrier may be a cloth or a sponge. The cuff may be made of materials that do not, or not substantially, absorb moisture. The substrate may be made from plastics, such as a coated plastics fabric (e.g., Nylon coated by polyurethane). The welds may be created by ultrasound welding. The complementary closure members may have no undercuts.

### Brief Description of the Drawings

Further details, objects and advantages of the present disclosure will become apparent from the detailed description which follows, and the accompanying drawings, in which
- Fig. 1: is top view of a first variant of a cuff in accordance with the present disclosure;
- Fig. 2: is a cross-sectional view along line A-A in Fig. 1;
- Fig. 3A: is an enlarged view of detail B of Fig. 2, illustrating in a cross-section view of attachment of a loop to a cuff substrate;
- Fig. 3B: is a perspective view of the loop of Fig. 3A;
- Fig. 3C: is a cross-sectional view of the loop of Fig. 3A in its length direction;
- Fig. 4: is a cross-sectional view along line E-E in Fig. 2, illustrating a front view of a web;
- Fig. 5: is an enlarged view of detail C of Fig. 2, illustrating an exemplary cross-sectional view of the web;
- Fig. 6: is an enlarged view of detail D of Fig. 1, illustrating a fluid port;
- Fig. 7: is top view of a second variant of a cuff in accordance with the present disclosure;
- Fig. 8: is a cross-sectional view along line A-A in Fig. 7;
- Fig. 9A: is an enlarged view of detail C of Fig. 8, illustrating a cross-sectional view of a web;
- Fig. 9B: is an enlarged view similar to Fig. 9A, illustrating a cross-sectional view of an alternative web design;
- Fig. 10: is an enlarged view of detail B of Fig. 8, illustrating a cross-sectional view a closure element cooperating with the loop attached to the cuff substrate;
- Fig. 11A: is a top view of the closure element illustrated in Figs. 7 and 10;
- Fig. 11B: is a side view of the closure element of Fig. 11A;
- Fig. 11C: is a bottom view of the closure element of Fig. 11A;
- Fig. 11D: is a cross-sectional view along line B-B of Fig. 11A;
- Fig. 11E: is an enlarged view of detail C of Fig. 11D, illustrating a channel for receiving the loop;
- Fig. 11F: is a cross-sectional view along line A-A of Fig. 11C, illustrating a chamber that is to receive a magnet;
- Fig. 12: is top view of a third variant of a cuff in accordance with the present disclosure;
- Fig. 13A: is a top view of an unfolded sheet of substrate material for manufacturing the various cuff variants presented herein, illustrating a window that is to receive the closure member carrying the webs;
- Fig. 13B: is a top view of the closure member that is to cover the window of the substrate material illustrated in Fig. 13A; and
- Fig. 14: is a flow diagram illustrating a method of using the cuff presented herein for blood pressure measurements.

### Detailed Description

In the following description, the same reference numerals are used to denote the same or similar components.

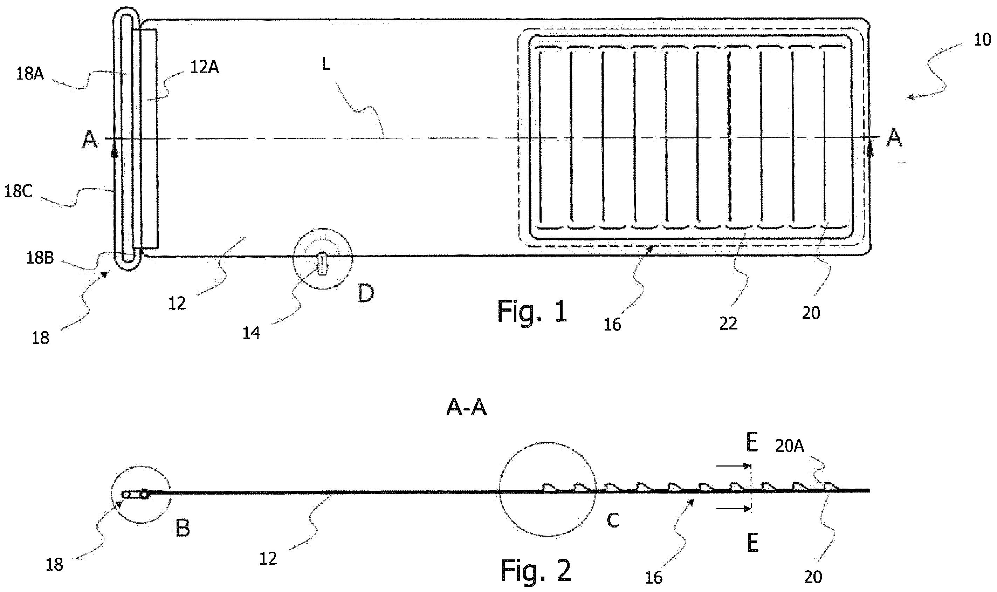

Fig. 1 is top view of a first variant of a cuff 10 in accordance with the present disclosure. Fig. 2 illustrates a cross-sectional view of the cuff 10 along line A-A in Fig. 1. The cuff 10 of Figs. 1 and 2 is configured for blood pressure measurements and, therefore, comprises a fluid chamber as will be described in greater detail below. In other variants, for example in case the cuff 10 is to be used as constricting band (also called tourniquet), the fluid chamber may be omitted.

As becomes apparent from Figs. 1 and 2, the cuff 10 has a flat, band-like shape with two free ends on opposite sides. The cuff 10 is configured to be wound around a person's limb for reducing or inhibiting a blood flow through the limb, as generally known in the art.

The cuff 10 comprises a flexible and substantially non-stretchable substrate 12 defining a substrate width, a substrate length and a longitudinal axis L extending along the substrate length. The substrate width may substantially be constant along the longitudinal axis L. The substrate 12 may have a width between 10 and 300 mm, preferably between 100 and 180 mm (e.g., between 120 and 150 mm for adults and in a configuration for blood pressure measurements), and a length between 80 and 600 mm, preferably between 180 and 360 mm (e.g., between 220 and 320 mm for adults and in a configuration for blood pressure measurements).

The substrate 12 is flexible in that it can be wound around a limb, but is substantially non-stretchable at least in a length direction parallel to the longitudinal axis L and, in a preferred implementation, also in a width direction perpendicular to the longitudinal axis L. As will be appreciated, substantial non-stretchability of the substrate 12 is an important prerequisite for proper blood pressure measurements as the substrate 12 must not, or at least not substantially, be stretchable when closed around a limb with the fluid chamber being inflated. Therefore, the substrate 12 in one implementation comprises a substantially non-stretchable fabric (i.e., textile) material, such as Textane A/B 70 (manufactured by SO.L.ER S.r.l.). A breaking strength (ISO 1621 CRT) of the fabric material may be above 250 N/5cm, preferably above 450 N/5cm, for both warp and weft, and an elongation at break (ISO 1621 CRT 4.3.2.e) may be below 40%, preferably below 30%, for warp and below 55%, preferably below 45%, for weft.

The fabric material may have a small mesh width. As will be appreciated, a smaller mesh width leads to a smoother substrate surface and facilitates a wipe disinfection. As an example, a roughness (root-mean-squared, rms, roughness) of the fabric material may be lower than 1000 nm, in particular lower than 700 nm or lower than 400 nm. The fabric material may comprise a woven thread. The thread may comprise or consist of a plastics material, such as Nylon (e.g., from 60 to 100 dtex, such as 78 dtex).

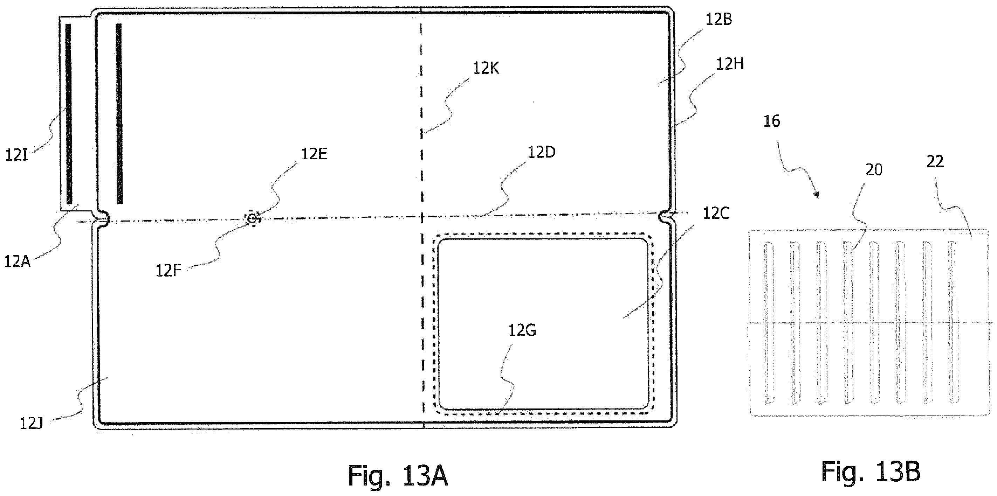

The fabric material of the substrate 12 may have an inner coating with a low gas permeability. Such a coating permits to define the fluid chamber between two layers of the coated fabric material, without there being any need for manufacturing a separate fluid chamber and arranging it for example in a pocket defined by the fabric material. As will be described in more detail below with reference to Figs. 13A and 13B below, a larger sheet of coated fabric material may be folded along a central line to define the two layers that form the fluid chamber between them.

The coating of the fabric material may comprise or consist of polyurethane (e.g., PU ether) that may be obtained by an extrusion process. The coating may be attached to the fabric material via an adhesive, such as PU or PU ether. The adhesive may be applied to the fabric material by a spreading process.

The cuff 10 illustrated in Figs. 1 and 2 is configured to be closed in a configuration in which it is wound around a person's limb. When the cuff 10 is closed, the fluid chamber can be inflated via a fluid port 14 to increase the pressure on the limb and so as to inhibit, or at least reduce, blood flow through the limb, without the cuff 10 deforming (i.e., expanding) in a direction radially away from the limb. In other words, in the closed state the cuff 10 withstands the pressure generated by the inflated fluid chamber (e.g., up to 300 mmHg), so that the fluid chamber is deformed in a direction radially towards the limb and pressure is exerted on the limb.

To close the cuff 10 around the limb, the cuff 10 comprises a closure mechanism with two cooperating closure members 16, 18. The two closure members 16, 18 are configured to engage each other via mutual abutment over an extended engagement zone that is smooth and closed. In the engagement zone there are no openings (e.g., windows, undercuts or textile loops) and no protrusions. Such openings or protrusions would be difficult to properly disinfect via wipe disinfection (e.g., using a cloth soaked with a disinfectant). In their abutment regions, a roughness (rms) of the two closure members 16, 18 may be below 700 nm, in particular below 500 nm or below 300 nm. In other words, their roughness may be lower than the roughness of the fabric material of the substrate 12. In some implementations, the fabric material may thus be the material of the cuff 10 with the highest roughness.

In the present implementation, the closure mechanism requires that a first closure member 16 is threaded through a slit-like opening, or eye, 18B of a second closure member 18 prior to a stable (i.e., force transmitting) final engagement of the two closure members 16, 18, that results from inflation of the fluid chamber. A threading-induced pre-engagement of the two closure members 16, 18 stabilizes the closure mechanism after the cuff 10 has been wound around the limb and before the fluid chamber is being inflated. Stability of the pre-engagement is increased by the fact that the first closure member 16 extends over substantially the entire width of the substrate 10 and that the eye 18B has a width that exceeds the width of the substrate 12. The eye 18B, or any similar opening in the second closure member 18, may have a length dimension that exceeds the width dimension of the cuff 10 in the region of the first closure member 16. The eye 18B, or any similar opening in the second closure member, may have a width dimension that is slightly larger (e.g., by 1, 2, 3, 4 or 5 mm) than a maximum thickness of the cuff 10 in the region of the first closure member 16 (as typically defined by a maximum height of the webs 20).

In certain implementations, the mutual abutment of the two closure members 16, 18 may be such that the closure members 16, 18 are not fixed against a possibly limited relative movement in a width direction perpendicular to the longitudinal axis L of the substrate 12 (e.g., over 1, 2, 3 or more mm), especially after the cuff 10 has been wound around the limb and before the fluid chamber is inflated (e.g., when the first closure member 16 is being or has been threaded through the second closure member 18). Such a relative movement facilitates engagement (and disengagement) of the closure mechanism as no exact, predefined relative position of the closure members 16, 18 in the width direction has to be found by the user.

With reference to Figs. 1 and 2, the first closure member 16 is attached to the substrate 12 and comprises a plurality of webs 20 extending parallel to each other and over substantially the entire width of the substrate 12. As shown in Figs. 1 and 2, the parallel webs 20 are spaced apart from each other along the longitudinal axis L of the substrate 12 and extend perpendicular to this longitudinal axis L. The spaced apart webs 20 define length increments at which the cuff 10 can be closed around the limb. These length increments can be 5 to 15 mm, e.g., 8 to 12 mm. The webs 20 are made from a plastics material such as PU or thermoplastic PU (TPU).

In typical configurations, the first closure member 16 comprises 5 to 15 (preferably 8 to 12) webs 20. The webs 20 have a linear extension. The webs 20 may have a length in a width direction of the substrate 12 that extends over at least 30%, 50% or 70% of the substrate width. The webs 20 may have a thickness at a bottom of 3 to 10 mm, preferably 4 to 8 mm, measured in a length direction of the substrate 12. The webs 20 may have a thickness at their top portion that is the same as, or smaller than, the thickness at their bottom portion. The webs 20 may have a height of 2 to 15 mm, preferably between 3 to 8 mm (e.g., 4, 5 or 6 mm), measured from its bottom.

The webs 20 can have any cross-sectional shape suitable to securely engage the complementary closure member 18. In some implementations, the webs 20 define an undercut on their face that is to be engaged by the complementary closure member 18, or a planar face that extends substantially perpendicular to the substrate 12 in a flat, non-wound configuration of the substrate 12. Exemplary cross-sectional shapes are discussed below. The webs 20 of the first closure member 16 may all have an identical shape or may have different shapes.

While the webs 20 are shown in Fig. 1 to have a continuous configuration with a constant cross-section along their lengths, in other implementations each web 20 may be discontinuous in the width direction of the substrate 12. As an example, a discontinuous web may be defined by two, three or more sub-webs aligned along the width direction of the substrate. The sub-webs may be spaced apart from each other in the width direction of the substrate 12 in a manner that allows an easy wipe disinfection between adjacent sub-webs (e.g., at a distance of at least 5 mm, 10 mm or more).

The closure member 16 further comprises a flexible planar basis 22 that extends at least between, and interconnects, the webs 20. The planar basis 22 has a surface that is at least one of continuous, closed und smooth between adjacent webs 22 so as to allow an easy wipe disinfection. In some implementations, the planar basis and the webs 20 form a structure (e.g., an integral structure) made from a plastics material (e.g., PU or TPU). The webs 20 may be welded to the planar basis 22 (e.g., using ultrasound welding). The planar basis 22 may have a rigidity that is low enough to allow an easy bending or flexing of the first closure member 16, but high enough to cause the first closure member 16 to elastically assume its planar shape when a bending or flexing force is removed.

In such or other configurations, the closure member 16 or at least a part thereof may be manufactured by injection molding, 3D-printing or otherwise. The planar basis 22, and thus the closure member 16, is attached to the substrate 12 at its circumference (as indicated by a dashed line in Fig. 1). In some implementations, the planar basis 22 is sized to close a window in the substrate 12, as will be explained below with reference to Figs. 13A and 13B.

With continued reference to Figs. 1 and 2, the second closure member 18 that engages the first closure member 16 is attached to the substrate 12 at a free end of the substrate 12. The second closure member 18 comprises two sections 18B, 18C that have a linear configuration and extend substantially parallel to each other. In the present implementation, the sections 18B, 18C define a closed loop forming the eye 18A. In other implementations, the two sections 18B, 18C could be connected at only one of their ends to form an open bracket. The loop or bracket may have a sufficient rigidness to not deform under the forces typically applied in use of the cuff 19. The loop or bracket can be manufactured from metal (e.g., steel) or any other material.

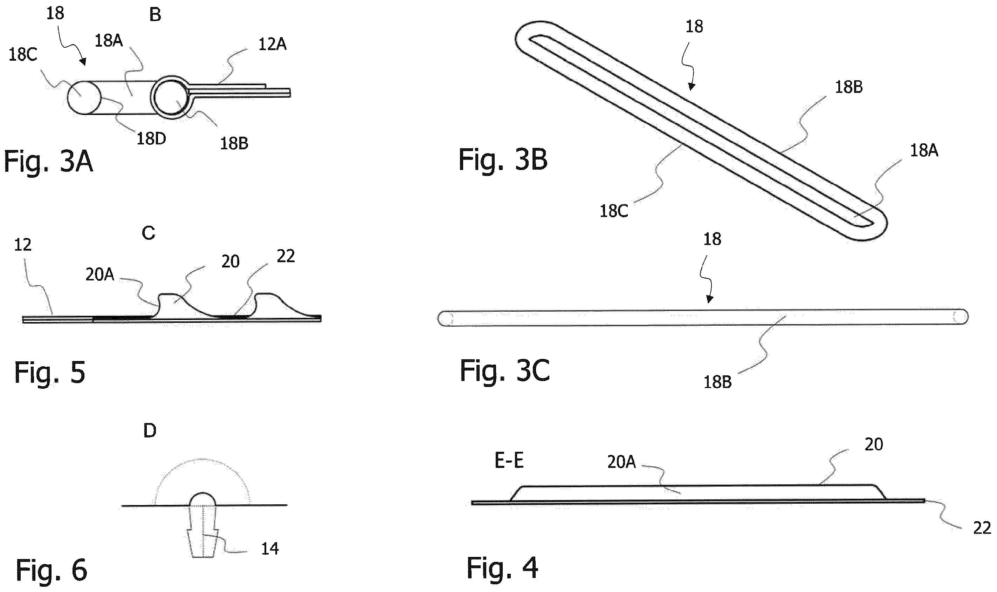

As becomes apparent from Fig. 1, a first one 18B of the two linear sections of the closure member 18 is pivotally attached to the substrate. Fig. 3A is an enlarged view of detail B of Fig. 2 and illustrates in a cross-sectional view how the closure member 18 is attached to the cuff substrate 12. As can be seen from Figs. 1 and 3A, a lateral end section 12A of the substrate 12 is folded over the linear section 18B of the closure member 18 to define a fold pivotally accommodating that linear section 18B. The folded over substrate end section 12A is attached to the body of the substrate 12, preferably by ultrasound welding, so that the closure member 18 cannot become loose while remaining pivotable in the fold. Other attachment techniques such as gluing could be used as well. Fig. 3B is a perspective view and Fig. 3C is a cross-sectional view of the closure member 18 in its length direction.

As mentioned above, the two closure members 16, 18 are configured to engage each other via mutual abutment over an extended engagement zone. The abutment in the closed configuration of the two closure members 16, 18 is such that the closure members 16, 18 cannot be disengaged once the fluid chamber has sufficiently been inflated, due to a force pressing the two closure members 16, 18 against each other into blunt abutment over the engagement zone. There is no further functional engagement of the two closure members 16, 18 outside of the engagement zone.

In general, the engagement zone can take the form of an engagement line or an engagement area. The engagement zone extends along a width direction of the cuff 10. The engagement zone, and thus the mutual abutment, can extend over more than 30%, 50% or 70% of the cuff width, which is typically defined by a width of the substrate 12. In this regard, Fig. 4 is a cross-sectional view of the first closure member 16 along line E-E in Fig. 2 and shows a front view of one of the webs 20. The web 20 is configured to be engaged by the complementary closure member 18 over its entire width. As becomes apparent from Figs. 1 and 4, the web 20 extends over substantially the entire width of the planar basis 22 and, thus, of the cuff 10.

With reference to Figs. 1 and 2, in the present variant the engagement zone consists of a first abutment region defined by a front face 20A of one of the webs 20 of the first closure member 16 and a complementary second abutment region 18D at the inside of the section 18C of the second closure member 18 (see Fig. 3A). The complementary abutment regions 18D, 20A are continuous and smooth, and they have no undercuts or openings. The complementary abutment regions 18D, 20A engage each other via a blunt abutment. As a result, the abutment regions 18D, 20A, and thus the entire closure mechanism, can easily be operated and easily be disinfected.

With reference to Figs. 1 and 5 (detail C of Fig. 2), the front face 20A of each web 20 is directed towards the second closure member 18 in a flat, laid-out configuration of the cuff 10. As becomes apparent from the cross-sectional view of Fig. 5, the front face 20A defines an undercut. In more detail, the front face 20A is concavely curved so as to accommodate the round cross-section of the abutment region 18D of linear section 18C of the second closure member 18, which has a circular cross-section. It is to be noted that the radii of curvature of the front face 20A and of the abutment region 18D may, but need not be substantially the same. In other words, there may, but need not be a form fitting abutment over the engagement zone. For example, the radius of curvature of the front face 20A may be larger than the radius of curvature of the abutment region 18D. Such a configuration particularly facilitates the engagement and disengagement of the two closure members 16, 18.

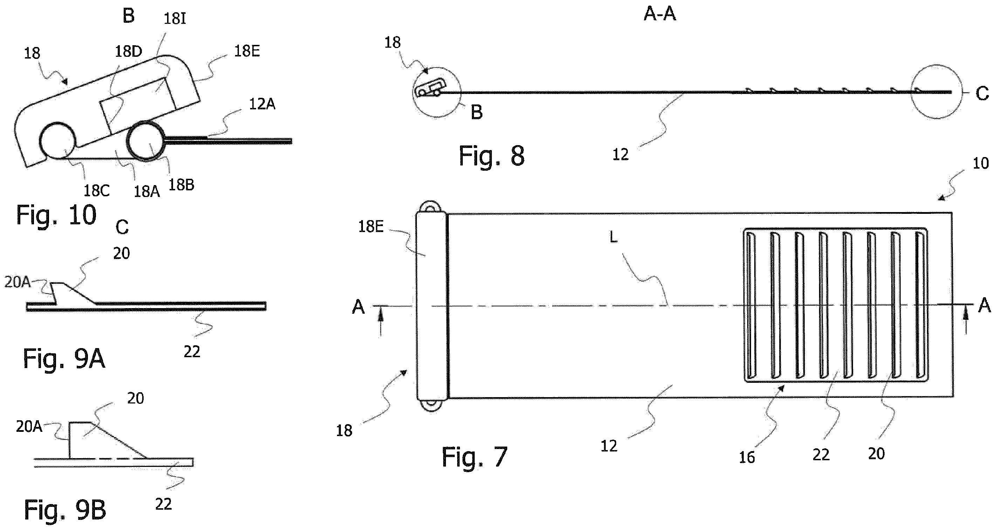

It is to be noted that the front faces 20A of the webs 20 do not necessarily have to have a concavely curved cross-section. The closure mechanism presented herein also works properly if the front face 20A defines an undercut (see Fig. 10A) or even a perpendicular surface relative to the substrate 12 in a flat configuration of the cuff (see Fig. 10B) in view of the deformations resulting in use. Similar considerations apply to the shape of the abutment region 18D of section 18C of the second closure member 18.

In use, the cuff 10 of Fig. 1 is wound around a limb such that the webs 20 face away from the limb. Then, the portion of the substrate 12 carrying the first closure member 16 is threaded through the eye 18A of the second closure member 18 (i.e., inserted between the two sections 18B, 18C of the second closure member 18) so as to form a loop around the limb. Threading of the first closure member 16 is facilitated by a sufficient rigidness of the planar basis 22 that prevents an undesired warping of the free end of the cuff 10 when being threaded through the eye 18A.

The first closure member 16 is threaded through the second closure member 18 until the inner diameter of the cuff 10 is slightly larger than the outer diameter of the limb, giving rise to a certain increase of the force required to pull at the free end of the cuff 10 opposite to the second closure member 18. When this pulling force is slightly decreased, the first closure member 16 will slip back through the second closure member 18 until section 18B of the second closure member 18 comes into an abutting pre-engagement with the last web 20 that has been threaded through the second closure member 18. Depending on the application of the cuff 10 to the limb, the pre-engagement may also comprise that the second closure member 18 is trapped between two adjacent webs 20 (e.g., that the section 18C can move only between an abutting relationship with either one of the two adjacent webs 20).

It is to be noted that operation of the cuff 10 does not require folding the portion of the substrate 12 carrying the first closure member 16 (and threaded through the eye 18A) over the section 18C, as is the case by conventional cuffs using a hook-and-loops-type closure mechanism. This fact facilitates operation of the cuff 10 presented herein.

In a next step, a fluid medium such as air is pumped through the fluid port 14 (see the detailed view of Fig. 6) to inflate the fluid chamber and increase the pressure on the limb. The resulting pressure increase on the limb causes a counter-force which forces section 18B of the second closure member 18 into firmer engagement with the front face 20A of the selected web 20 due to a force component that acts in a circumferential direction of the cuff 10 and results in a stable final engagement that prevents the closure mechanism from becoming inadvertently disengaged.

Once the desired pressure in the fluid chamber has been reached, the fluid is permitted to slowly exit the fluid chamber through the fluid port 14, while measuring the blood pressure in a known manner using a manometer or similar measuring device. After a full deflation of the fluid chamber, the pressure on the limb and the force acting on the closure mechanism are reduced to an extent that allows to unthread the portion of the substrate 12 carrying the first closure member 16 through the second closure member 18 and remove the cuff 10 from the patient's limb.

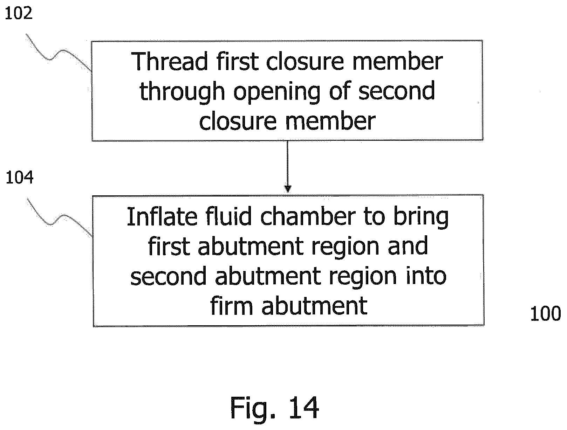

The other cuff variants presented herein will be operated in substantially the same manner. Further details of using the cuff 10 will be described below with reference to Fig. 14.

Fig. 7 is top view of a second variant of a cuff 10 in accordance with the present disclosure, and Fig. 8 is a cross-sectional view along line A-A in Fig. 7. The second cuff variant is to a large extent identical to the first cuff variant described above with reference to Figs. 1 to 6. For this reason, the following description of the second cuff variant will mainly concentrate on the differences. While not explicitly shown, the second cuff variant also comprises a fluid port in communication with a fluid chamber (see Fig. 6).

One difference between the first cuff variant and the second cuff variant is the fact that the webs 20 can have a different cross-section. Fig. 9A is enlarged view of detail C of Fig. 8, illustrating a cross-sectional view of one of the webs 20. As becomes apparent from Fig. 9A, the front face 20A defining the abutment region has an undercut. In more detail, the front face 20A is defined as a planar surface that extends from the planar substrate 22 (in a flat configuration of the substrate 12) at an angle of less than 90° and more than 45° (e.g., at an angle above 65° or 75° and below 85° or 80°). Fig. 9B is enlarged view similar to Fig. 9A, illustrating a cross-sectional view of an alternative web design. As becomes apparent from Fig. 9B, the front face 20A is again defined as a planar surface that extends from the planar substrate 22 (in a flat configuration of the substrate 12), but now at an angle of 90°.

A further difference compared the first cuff variant of Figs. 1 to 6 resides in the fact that the closure member 18 of the second cuff variant further comprises a closure element 18E pivotally supported by the section 18C of the loop (or bracket) that forms the closure member 18 of the first cuff variant (see Figs. 3A and 3B).

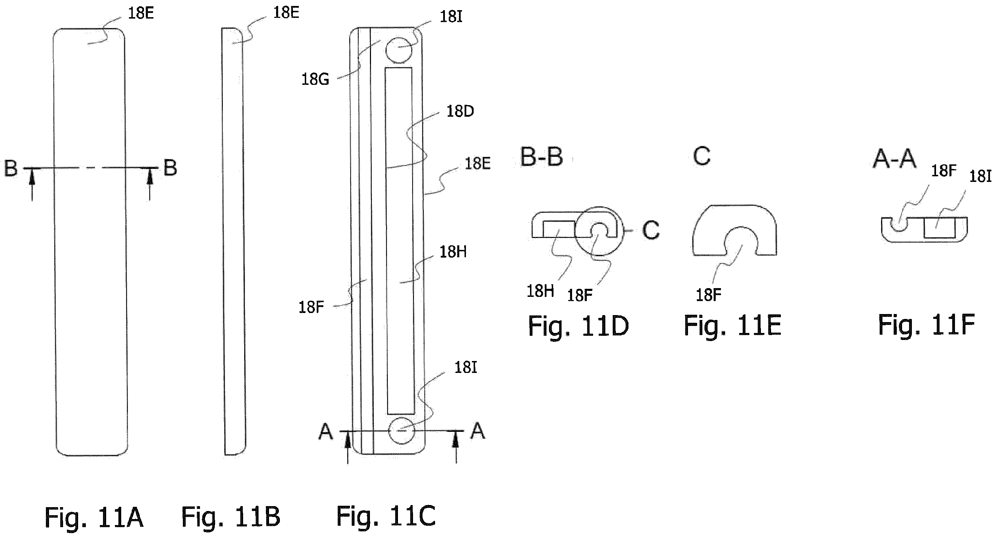

Fig. 10 is enlarged view of detail B of Fig. 8, illustrating in a cross-sectional view the pivotable closure element 18E cooperating with the loop attached to the cuff substrate 12. Fig. 11A is a top view of the closure element 18E, Fig. 11B is a side view of the closure element 18E, Fig. 11C is a bottom view of the closure element 18E, Fig. 11D is a cross-sectional view along line B-B of Fig. 11A, Fig. 11E is an enlarged view of detail C of Fig. 11D, and Fig. 11F is a cross-sectional view along line A-A of Fig. 11C.

With reference to Fig. 10, the closure element 18E is configured to define an abutment region 18D that is to engage the complementary abutment region (i.e., the front face 20A) of one of the webs 20 when the closure element 16 has been threaded through the eye 18A of the closure member 18. Compared to the first cuff variant, the abutment region 18D of the closure member 18 is thus no longer defined by a surface of the linear section 18C (see Fig. 3A), but by an internal surface of the closure element 18E pivotally supported by the linear section 18C (see Fig. 10).

As becomes apparent from Figs. 10, 11C, 11D and 11E, the closure element 18E comprises an open channel 18F at its bottom surface 18G. The channel 18F extends over the entire length of the closure element 18E and opens towards the bottom and side faces of the closure element 18E. The channel 18F has a circular cross-section with a diameter that substantially corresponds to the diameter of the circular cross-section of the linear section 18C (see Fig. 10). The channel 18F is closed over more than 200° but less than 300°, so that the linear section 18C can be snapped into the channel 18F for a form-fitting, pivotable engagement thereof.

As becomes apparent from Figs. 10, 11C and 11D, the closure element 18E further comprises a groove 18H at its bottom surface 18G. The groove 18H has a surface adjacent to the bottom surface 18G, and this surface defines the abutment region 18D. In the present example, the groove 18H has a rectangular cross-section, so that its surface defining the abutment region 18D is planar and extends perpendicular to the bottom surface 18G. In use, the abutment region 18D defines an undercut that engages the front face 20A of one of the webs 20. The planar abutment region 18D with its perpendicular extension to the bottom surface 18G ensures an easy engagement and disengagement with and from a particular web 20. In some implementations, the abutment region 18D could have an oblique extension relative to the bottom surface 18G, such that a width of the groove 18H is larger at the groove bottom than at the open side of the groove 18H. In such or other implementations, the groove 18G could also be open at its side opposite the abutment region 18D.

With reference to Fig. 11C, the groove 18G does not extend over the entire length of the closure member 18E. Rather, it extends over a substantial portion thereof (e.g., over more than 30%, 50% or 70%), but is closed at its opposite ends. Adjacent to the opposite ends of the groove 18G, the bottom surface 18G is provided with two chambers (here: of circular cross-section) that each accommodates a magnet 18I. The magnets 18I are configured to engage the (metallic) section 18B (see Fig. 10) when the closure element 18E is pivotally closed over the closure member 16 threaded through the eye 18A. In this configuration, the magnets 18I attract the section 18B of the closure member 18 and ensure a stable pre-engagement of the complementary abutment regions 18D, 20A prior to inflation of the fluid chamber. It will be appreciated that the magnets 20I are optional. A suitably selected friction fit between the linear section 18C and the channel 18F could serve a similar purpose.

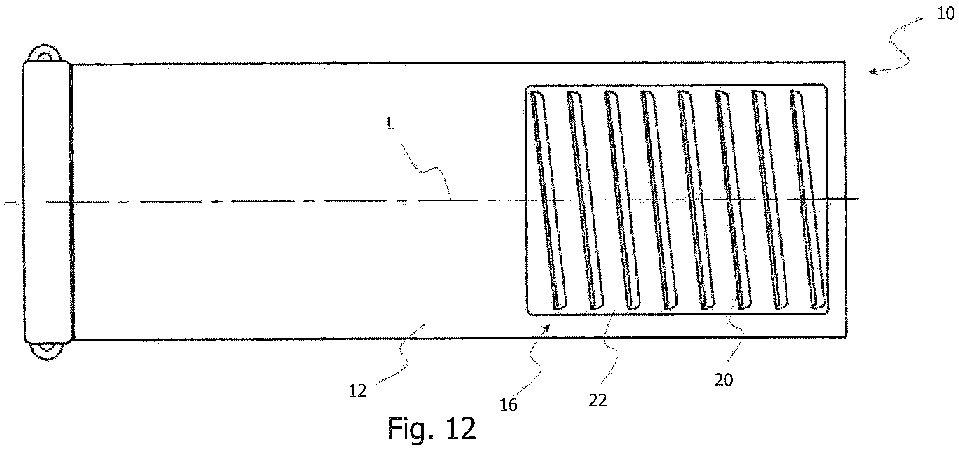

Fig. 12 is top view of a third variant of a cuff 10 in accordance with the present disclosure. The third cuff variant is to a large extent identical to the second cuff variant described above with reference to Figs. 7 to 11F. For this reason, the following description of the third cuff variant will concentrate on the differences between these to variants.

As becomes apparent from Fig. 12, the webs 20 of the cuff 10 do not extend perpendicular relative to the longitudinal axis L of the substrate 12, but have an oblique extension. An angle between the longitudinal extension of the webs 20 and the longitudinal axis L of the substrate 12 may be selected below 90° and above 45° (e.g., between 60° and 85°).

An oblique extension of the webs 20 results in a conical shape of the cuff 10 in a closed configuration. Since a limb generally also has a conical shape tapering in a direction away from the body, the oblique webs 20 ensure that the closed cuff 10 better conforms to the shape of the limb. Of course, oblique webs 20 can also be implemented for the cuff 10 of the first variant (see Figs. 1 to 6) or any other cuff variant.

The cuffs 10 presented herein can be manufactured in many different ways. In the following, an exemplary manufacturing process will be described with reference to Figs. 13A and 13B.

The manufacturing process comprises folding a larger pre-shaped sheet of substrate material onto itself. The substrate material may be comprise a coated fabric as generally described above.

Fig. 13A is a top view of a sheet 12B of substrate material of the cuff 10 in an unfolded state. As becomes apparent from Fig. 13A, the sheet 12B has a window 12C that is to receive the closure member 16 with the planar base 22 carrying the webs 20. Fig. 13B is a top view of the closure member 16 that is to cover the window 12C of the substrate material illustrated in Fig. 13A (here in the configuration also illustrated in Fig. 7).

Fig. 13A illustrates a folding line 12D along which the sheet 12B will be folded onto itself. On the folding line 12D lies a circular opening 12E that is to receive the fluid port 14 (see Fig. 6). Prior to the folding step, the fluid port 14 is inserted into the opening 12E and attached to the sheet 12B, for example by ultrasound welding along the dashed line 12F.

Still prior to the folding step, the closure member 16 is placed on the sheet 12B to close the window 12C. The outer dimensions of the planar basis 22 are slightly larger than the dimensions of the window 12C in the sheet 12B, so that an outer rim of the planar basis 22 overlaps with a portion of the sheet 12B adjacent to the window 12C when the webs 20 reach through the window. In a next step, the closure member 16 is attached to the sheet 12C, for example by ultrasound welding along a welding line 12D extending along the rim of the planar basis 22. The resulting weld will be gap-free and allow for a save wipe disinfection (e.g., compared to stitching the closure member 16 to the sheet 12B).

Then, the sheet 12B is folded onto itself along the folding line 12D so that the folded portion of the sheet 12B rests on the backside of the planar basis 22 (opposite the webs 20), with the overlapping rim of the planar basis 22 being sandwiched between the resulting two layers of the sheet 12B. In a further ultrasound welding step along a welding line 12H running around the three free sides of the folded sheet 12B, a fluid-tight inner space is created between the two layers of the sheet 12B folded onto each other. The fluid-tightness results from the coating of the fabric material from which the sheet 12B is made. The coating is provided on the side of the sheet 12B that is folded onto itself.

In a further manufacturing step, the lateral end section 12A is folded over the linear section 18B of the closure member 18 to define a fold pivotally accommodating that linear section 18B (see Figs. 3A and 10). The fold is stabilized by ultrasound welding along a welding line 12I. The resulting weld will be gap-free and allow for a save wipe disinfection.

Next, a fluid chamber 12J is defined by an ultrasound step along a welding line 12K. As becomes apparent from Fig. 13A, the fluid chamber 12J is defined such that it includes the portion of the folded sheet 12B in which the circular opening 12E (with the fluid port 14 extending therethrough) is provided. The fluid chamber 12J, on the other hand, is fluidically separated from the portion of the folded sheet 12B to which the closure member 16 is attached.

For the variant of the cuff 10 illustrated in Figs. 7 to 11F, the closure member 18E is then attached to the free linear section 18C of the closure member 18. To this end, the section 18C is snapped into the open channel 18F provided in the bottom surface 18G of the closure member (see Figs. 10 and 11D).

Fig. 14 is a flow diagram 100 illustrating a method of using the cuff 10 of any of the variants presented herein, or other variants, for blood pressure measurements.

In a first step 102, the first closure member 16, with the webs 20 facing radially outwardly, is threaded through an opening of the second closure member 18, such as the exemplary eye 18A. As a result, the cuff 10 will form a loop. The threading step 102 may occur before or after applying the cuff 10 to a limb, such as an arm.

Once the cuff 10 is applied to the limb, the threading may continue until the inner diameter of the cuff 10 is slightly larger than the outer diameter of the limb and a counter-force starts to act. When the threading force is then reduced, the first closure member 16 may slightly slip back through the eye 18A until the abutment region of the first closure member, i.e., the front face 20A of one of the webs 20, is caught by, and thus comes into blunt abutment with, the complementary abutment region 18D of the second closure member 18. For the second variant of the cuff 10 discussed above with reference to Figs. 7 to 11F, the closure member 18E first needs to be pivoted towards the linear section 18B for the abutment region 18D to catch the front face 20A of one of the webs 20. This pivoted configuration of the closure member 18E is maintained by the magnetic force of the magnets 18I acting on the linear section 18B.

After a pre-engagement between the complementary abutment regions 18D, 20A has been achieved as outlined above, the fluid chamber 12J is inflated. As a result of the inflation, the cuff 10 will deform radially inwardly and the inner diameter of the cuff 10 will decrease and firmly press against the limb to reduce or inhibit a blood flow. The resulting counter-force will act radially outwardly on the cuff 10 and, since the cuff 10 cannot expand, result in a force acting in a circumferential direction of the cuff 10. This circumferential force will bring the abutment regions 18D, 20A into a firmer abutment (see step 104 of Fig. 14), so that the two closure members 16, 18 securely engage each other and prevent the cuff 10 from becoming loose. In more detail, the circumferential force will pull on the pivotably supported second closure member 18 and thus rotate it around the linear section 18B in a stable force-transmitting position relative to the face 20A of the web 20.

Then, the fluid chamber 12J can continuously be deflated for blood pressure measurements, as generally known in the art. Once the fluid chamber 12J has fully been deflated, there is no more pressure acting on the limb and, thus, no more circumferential force pressing the two closure members 16, 18 into firm engagement. Consequently, the first closure member 16 can be threaded back through the eye 18A of the second closure member 18. For the second variant of the cuff 10 discussed above with reference to Figs. 7 to 11F, the closure member 18E first needs to be pivoted away from the linear section 18B so that the front face 20A previously engaged by the abutment region 18D becomes free and can slip through the eye 18A.

It should be noted that a threadable configuration of the cuff 10 is advantageous in regard to achieving an at least partially stable pre-engage of the two closure members 16, 18, but that non-threadable configurations of the closure mechanism may be provided as well.

As has become apparent from the above description, the cuff 10 can easily be operated by a user. Moreover, the cuff 10 permits a safe wipe disinfection as it only comprises closed smooth surfaces that can easily be reached by a cloth soaked with a disinfectant or by any other soft carrier of a disinfectant, such as a sponge. The effectiveness of disinfecting surfaces by wipe disinfection depends on the duration the disinfectant can act on the cuff surfaces and the mechanical aspects of sufficient pressure and reaching all surfaces. The lack of openings, in particular the gap-free welds by which the closure members 16, 18 are attached to the substrate 12, and the smooth surfaces of the cuff 10, in particular at the abutment regions of the closure members 16, 18, thus greatly facilitate a wipe disinfection (e.g., in less than 60 seconds).

While the variants of the cuff 10 described above thus are advantageous both in regard of operability and sterility, other variants may provide only one of these two advantages.

The cuff 10 may be a re-usable item or configured for single use. Since the cuff 10 can be manufactured solely from Nylon and PU (or TPU), it can easily be recycled.

## Claims

1. A cuff (10) configured to be closed around a person's limb for reducing or inhibiting a blood flow through the limb, the cuff (10) comprising:
a flexible and substantially non-stretchable substrate (12) defining a substrate length and a longitudinal axis (L) extending along the substrate length;
a first closure member (16) attached to the substrate (12), the first closure member (16) comprising a plurality of webs (20) spaced apart from each other along the longitudinal axis (L) of the substrate (12), each web (20) extending away from the substrate (12) and defining a first abutment region (20A) extending over at least 50% of a length of the web (20); and
a second closure member (18) attached to the substrate (12) at a free end thereof, wherein the second closure member (18) defines a second abutment region (18D) configured to cooperate with the first abutment region (20A) of one of the webs (20) of the first closure member (16) to close the cuff (10) around the limb, wherein
the second closure member (18) comprises two sections (18B, 18C) extending substantially parallel to each other and defining an opening (18A) therebetween, wherein a first one (18B) of the two sections (18B, 18C) is pivotally attached to the substrate (12).

2. The cuff (10) of claim 1, wherein
the first abutment region (20A) and the second abutment region (18D) are configured with a closed smooth surface.

3. The cuff (10) of any of the preceding claims, wherein
the second closure member (18) comprises the opening (18A) for threading the first closure member (16) through the second closure member (18).

4. The cuff (10) of claim 3, wherein
the cuff (10) permits the first abutment region (20A) and the second abutment region (18D) to come into abutment when first closure member (16) is threaded through opening (18A) of the second closure member (18).

5. The cuff (10) of any of the preceding claims, wherein
at least one of the first abutment region (20C) and the second abutment region (18D) extends over at least 30%, and in particular over at least 50% of a width of at least one of the substrate (12) and the cuff (10).

6. The cuff (10) of any of the preceding claims, wherein
a surface region of the cuff (10) between two adjacent webs (20) is closed.

7. The cuff (10) of any of the preceding claims, wherein
the first closure member (18) further comprises a planar basis (22) extending at least between two adjacent webs (20), wherein the basis (22) is attached to the substrate (12).

8. The cuff of claims 6 and 7, wherein
the planar basis (22) defines the closed surface region of the cuff (10) between two adjacent webs (20).

9. The cuff of claim 7 or 8, wherein
the substrate (12) comprises a substrate window (12C), and wherein the planar basis (22) closes the substrate window (12C).

10. The cuff of any one of claims 7 to 9, wherein
the planar basis (22) is attached to the substrate (12) by ultrasound welding.

11. The cuff (10) of any one of the preceding claims, wherein
the substrate (12) is made from a fabric material and the first closure member (16) is made from a plastics material.

12. The cuff (10) of any of the preceding claims, wherein
the second closure member (18) comprises a loop or bracket.

13. The cuff (10) of any of the preceding claims, wherein
a second one (18C) of the two sections (18B, 18C) defines the second abutment region (18D).

14. The cuff (10) of any of claims 1 to 12, wherein
a second (18D) one of the two sections (18B 18C) pivotally supports a closure element (18E) that defines the second abutment region (18D).

15. The cuff (10) of claim 14, wherein
the closure element (18E) comprises an undercut defining the second abutment region (18D).

16. The cuff (10) of any one of the preceding claims, wherein
the webs (20) extend non-perpendicular to the longitudinal axis (L) of the substrate (12).

17. The cuff (10) of any one of the preceding claims, wherein
the cuff (10) comprises an inflatable fluid chamber (12J).

18. A method of operating the cuff (10) of claims 3 and 17, or any one of the preceding claims when depending on claims 3 and 17, wherein the method comprises:
threading (102) the first closure member (16) through the opening (18A) of the second closure member (18); and
inflating (104) the fluid chamber (12J) to bring the first abutment region (20A) and the second abutment region (18D) into firm abutment.

19. A method of disinfecting the cuff (10) of any of claims 1 to 17, wherein the cuff (10) comprises a substrate (12) and two complementary closure members (16, 18) attached to the substrate (12) by gap-free welds (12G, 12I) and configured to close the cuff (10) around a person's limb by mutual abutment for reducing or inhibiting a blood flow through the limb, wherein the cuff (10) has smooth surfaces in abutting regions of the closure member (16, 18), wherein the method comprises:
wiping over the cuff (10) with a soft carrier of a disinfectant material.

## Patentansprüche

1. Manschette (10), die so ausgebildet ist, dass sie um die Extremität einer Person geschlossen werden kann, um den Blutfluss durch die Extremität zu verringern oder zu unterbinden, wobei die Manschette (10) umfasst:
ein flexibles und im Wesentlichen nicht dehnbares Substrat (12), das eine Substratlänge und eine Längsachse (L) definiert, die sich entlang der Substratlänge erstreckt;
ein erstes Verschlusselement (16), das an dem Substrat (12) befestigt ist, wobei das erste Verschlusselement (16) eine Vielzahl von Stegen (20) umfasst, die entlang der Längsachse (L) des Substrats (12) voneinander beabstandet sind, wobei sich jeder Steg (20) vom Substrat (12) weg erstreckt und einen ersten Anlagebereich (20A) definiert, der sich über mindestens 50 % einer Länge des Stegs (20) erstreckt; und
ein zweites Verschlusselement (18), das an dem Substrat (12) an einem freien Ende davon befestigt ist, wobei das zweite Verschlusselement (18) einen zweiten Anlagebereich (18D) definiert, der so ausgebildet ist, dass er mit dem ersten Anlagebereich (20A) eines der Stege (20) des ersten Verschlusselements (16) zusammenwirkt, um die Manschette (10) um die Extremität herum zu schließen, wobei
das zweite Verschlusselement (18) zwei Abschnitte (18B, 18C) umfasst, die sich im Wesentlichen parallel zueinander erstrecken und zwischen sich eine Öffnung (18A) bilden, wobei ein erster (18B) der beiden Abschnitte (18B, 18C) schwenkbar am Substrat (12) befestigt ist.

2. Manschette (10) nach Anspruch 1, wobei
der erste Anlagebereich (20A) und der zweite Anlagebereich (18D) mit einer geschlossenen, glatten Oberfläche ausgebildet sind.

3. Manschette (10) nach einem der vorstehenden Ansprüche, wobei
das zweite Verschlusselement (18) die Öffnung (18A) zum Durchfädeln des ersten Verschlusselements (16) durch das zweite Verschlusselement (18) umfasst.

4. Manschette (10) nach Anspruch 3, wobei
die Manschette (10) es ermöglicht, dass der erste Anlagebereich (20A) und der zweite Anlagebereich (18D) in Anlage kommen, wenn das erste Verschlusselement (16) durch die Öffnung (18A) des zweiten Verschlusselements (18) geführt ist.

5. Manschette (10) nach einem der vorstehenden Ansprüche, wobei
sich mindestens einer des ersten Anlagebereichs (20C) und des zweiten Anlagebereichs (18D) über mindestens 30% und insbesondere über mindestens 50% einer Breite von mindestens einem des Substrats (12) und der Manschette (10) erstreckt.

6. Manschette (10) nach einem der vorstehenden Ansprüche, wobei
ein Oberflächenbereich der Manschette (10) zwischen zwei benachbarten Stegen (20) geschlossen ist.

7. Manschette (10) nach einem der vorstehenden Ansprüche, wobei
das erste Verschlusselement (18) ferner eine ebene Basis (22) umfasst, die sich zumindest zwischen zwei benachbarten Stegen (20) erstreckt, wobei die Basis (22) an dem Substrat (12) befestigt ist.

8. Manschette nach den Ansprüchen 6 und 7, wobei
die ebene Basis (22) den geschlossenen Oberflächenbereich der Manschette (10) zwischen zwei benachbarten Stegen (20) definiert.

9. Manschette nach Anspruch 7 oder 8, wobei
das Substrat (12) ein Substratfenster (12C) umfasst und wobei die ebene Basis (22) das Substratfenster (12C) verschließt.

10. Manschette nach einem der Ansprüche 7 bis 9, wobei
die ebene Basis (22) durch Ultraschallschweißen am Substrat (12) befestigt ist.

11. Manschette (10) nach einem der vorstehenden Ansprüche, wobei
das Substrat (12) aus einem Gewebematerial und das erste Verschlusselement (16) aus einem Kunststoffmaterial gefertigt ist.

12. Manschette (10) nach einem der vorstehenden Ansprüche, wobei
das zweite Verschlusselement (18) eine Schlaufe oder einen Bügel umfasst.

13. Manschette (10) nach einem der vorstehenden Ansprüche, wobei
ein zweiter (18C) der beiden Abschnitte (18B, 18C) den zweiten Anlagebereich (18D) bildet.

14. Manschette (10) nach einem der Ansprüche 1 bis 12, wobei
ein zweiter (18D) der beiden Abschnitte (18B, 18C) ein Verschlusselement (18E) schwenkbar trägt, das den zweiten Anlagebereich (18D) definiert.

15. Manschette (10) nach Anspruch 14, wobei
das Verschlusselement (18E) eine Hinterschneidung aufweist, die den zweiten Anlagebereich (18D) definiert.

16. Manschette (10) nach einem der vorstehenden Ansprüche, wobei
sich die Stege (20) nicht senkrecht zur Längsachse (L) des Substrats (12) erstrecken.

17. Manschette (10) nach einem der vorstehenden Ansprüche, wobei
die Manschette (10) eine aufblasbare Fluidkammer (12J) umfasst.

18. Verfahren zum Betreiben der Manschette (10) nach den Ansprüchen 3 und 17 oder nach einem der vorstehenden Ansprüche, sofern dieser von den Ansprüchen 3 und 17 abhängt, wobei das Verfahren umfasst:
Einfädeln (102) des ersten Verschlusselements (16) durch die Öffnung (18A) des zweiten Verschlusselements (18); und
Aufblasen (104) der Fluidkammer (12J), um den ersten Anlagebereich (20A) und den zweiten Anlagebereich (18D) in feste Anlage zu bringen.

19. Verfahren zum Desinfizieren der Manschette (10) nach einem der Ansprüche 1 bis 17, wobei die Manschette (10) ein Substrat (12) und zwei komplementäre Verschlusselemente (16, 18) umfasst, die durch spaltfreie Schweißnähte (12G, 12I) am Substrat (12) befestigt und so ausgebildet sind, dass sie die Manschette (10) durch gegenseitige Anlage um die Extremität einer Person schließen, um den Blutfluss durch die Extremität zu verringern oder zu unterbinden, wobei die Manschette (10) in den anliegenden Bereichen des Verschlusselements (16, 18) glatte Oberflächen aufweist, wobei das Verfahren umfasst:
Abwischen der Manschette (10) mit einem weichen Träger eines Desinfektionsmaterials.

## Revendications

1. Brassard (10) conçu pour renfermer un membre d'une personne afin de réduire ou d'empêcher un écoulement sanguin dans ledit membre, le brassard (10) comprenant:
un substrat souple et sensiblement non extensible (12) définissant une longueur de substrat et un axe longitudinal (L) s'étendant sur la longueur du substrat;
un premier élément de fermeture (16) fixé au substrat (12), le premier élément de fermeture (16) comprenant une pluralité de nervures (20) espacées les unes des autres le long de l'axe longitudinal (L) du substrat (12), chaque nervure (20) s'étendant dans le prolongement du substrat (12) et définissant une première zone de butée (20A) s'étendant sur au moins 50 % de la longueur de la nervure (20); et
un second élément de fermeture (18) fixé au substrat (12) au niveau d'une extrémité libre de ce dernier, le second élément de fermeture (18) définit une seconde zone de butée (18D) conçue pour coopérer avec la première zone de butée (20A) de l'une des nervures (20) du premier élément de fermeture (16) afin de fermer le brassard (10) autour du membre, dans lequel
le second élément de fermeture (18) comprend deux sections (18B, 18C) s'étendant sensiblement parallèlement l'une à l'autre et définissant une ouverture (18A) entre elles, dans lequel une première (18B) des deux sections (18B, 18C) est fixée en pivotement au substrat (12).

2. Brassard (10) selon la revendication 1, dans lequel
la première zone de butée (20A) et la seconde zone de butée (18D) présentent une surface lisse et fermée.

3. Brassard (10) selon l'une quelconque des revendications précédentes, dans lequel
le second élément de fermeture (18) comporte une ouverture (18A) permettant d'enfiler le premier élément de fermeture (16) dans le second élément de fermeture (18).

4. Brassard (10) selon la revendication 3, dans lequel
le brassard (10) permet à la première zone de butée (20A) et à la seconde zone de butée (18D) de venir en butée lorsque le premier élément de fermeture (16) est enfilé dans l'ouverture (18A) du second élément de fermeture (18).

5. Brassard (10) selon l'une quelconque des revendications précédentes, dans lequel
au moins l'une parmi la première zone de butée (20C) et la seconde zone de butée (18D) s'étend sur au moins 30 %, et en particulier sur au moins 50 %, de la largeur d'au moins l'un parmi le substrat (12) et le brassard (10).

6. Brassard (10) selon l'une quelconque des revendications précédentes, dans lequel
une zone de surface du brassard (10) entre deux nervures adjacentes (20) est fermée.

7. Brassard (10) selon l'une quelconque des revendications précédentes, dans lequel
le premier élément de fermeture (18) comprend en outre une base plane (22) s'étendant au moins entre deux nervures adjacentes (20), la base (22) étant fixée au substrat (12).

8. Brassard (10) selon la revendication 6 ou 7, dans lequel
la base plane (22) délimite la zone de surface fermée du brassard (10) située entre deux nervures adjacentes (20).

9. Brassard (10) selon la revendication 7 ou 8, dans lequel
le substrat (12) comprend une fenêtre de substrat (12C), et dans lequel la base plane (22) ferme la fenêtre de substrat (12C).

10. Brassard (10) selon l'une quelconque des revendications 7 à 9, dans lequel
la base plane (22) est fixée au substrat (12) par soudage par ultrasons.

11. Brassard (10) selon l'une quelconque des revendications précédentes, dans lequel
le substrat (12) est composé d'un matériau en tissu et le premier élément de fermeture (16) est composé d'un matériau en plastique.

12. Brassard (10) selon l'une quelconque des revendications précédentes, dans lequel
le second élément de fermeture (18) comprend une boucle ou une patte.

13. Brassard (10) selon l'une quelconque des revendications précédentes, dans lequel
la seconde (18C) parmi les deux sections (18B, 18C) délimite la seconde zone de butée (18D).

14. Brassard (10) selon l'une quelconque des revendications 1 à 12, dans lequel
la seconde (18D) parmi les deux sections (18B, 18C) supporte en pivotement un élément de fermeture (18E) qui délimite la seconde zone de butée (18D).

15. Brassard (10) selon la revendication 14, dans lequel
l'élément de fermeture (18E) comprend une contre-dépouille délimitant la seconde zone de butée (18D).

16. Brassard (10) selon l'une quelconque des revendications précédentes, dans lequel
les nervures (20) ne sont pas perpendiculaires à l'axe longitudinal (L) du substrat (12).

17. Brassard (10) selon l'une quelconque des revendications précédentes, dans lequel
le brassard (10) comprend une chambre gonflable à fluide (12J).

18. Procédé de mise en œuvre du brassard (10) selon les revendications 3 et 17, ou selon l'une quelconque des revendications précédentes lorsqu'elle dépend des revendications 3 et 17, dans lequel le procédé comprend:
l'enfilement (102) du premier élément de fermeture (16) dans l'ouverture (18A) du second élément de fermeture (18); et
le gonflage (104) de la chambre à fluide (12J) afin de mettre la première zone de butée (20A) et la second zone de butée (18D) en contact étroit.

19. Procédé de désinfection du brassard (10) selon l'une quelconque des revendications 1 à 17, dans lequel le brassard (10) comprend un substrat (12) et deux éléments de fermeture complémentaires (16, 18) fixés au substrat (12) par des soudures sans interstice (12G, 12I) et conçus pour fermer le brassard (10) autour d'un membre d'une personne par butée réciproque afin de réduire ou d'empêcher l'écoulement sanguin dans le membre, dans lequel le brassard (10) présente des surfaces lisses dans les zones de butée des éléments de fermeture (16, 18), dans lequel le procédé comprend:
l'essuyage du brassard (10) à l'aide d'un support souple imprégné d'un produit désinfectant.
